(19) Europäisches Patentamt
European Patent Office
Office européen des brevets

(11) Numéro de publication: **0 398 818 A1**

# (12) DEMANDE DE BREVET EUROPEEN

(21) Numéro de dépôt: **90420179.5**

(22) Date de dépôt: **10.04.90**

(51) Int. Cl.5: **C07C 323/07, C07C 317/10, C07D 213/32, A01N 31/04, A01N 41/10, A01N 43/40**

(30) Priorité: **10.04.89 FR 8904940**

(43) Date de publication de la demande: **22.11.90 Bulletin 90/47**

(84) Etats contractants désignés: **AT BE CH DE DK ES FR GB GR IT LI LU NL SE**

(71) Demandeur: **RHONE POULENC AGROCHIMIE**
**14-20 rue Pierre-Baizet**
**F-69009 Lyon(FR)**

(72) Inventeur: **Desbordes, Philippe**
**30 Rue Bancel**
**F-69007 Lyon(FR)**
Inventeur: **Euvrard, Michel**
**33 Route du Mont Thou, Saint Romain au Mont D'or**
**F-69270 Fontaines sur Saone(FR)**
Inventeur: **De Reinach Hirtzbach, François**
**5 Quai Général Sarrail**
**F-69006 Lyon(FR)**
Inventeur: **Pearson, Christopher**
**7 Byde Street**
**Benge, Hertford, Hertfordshire, SG14 3AL(GB)**

(74) Mandataire: **Ranguis, Patrick Frédéric et al**
**RHONE POULENC AGROCHIMIE Service DPI**
**B.P. 9163**
**F-69263 Lyon Cédex 09(FR)**

## (54) Sulfones herbicides dihologénées.

(57) Composés de formule :

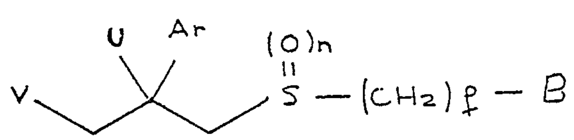

I

dans laquelle :
n = 0, 1, 2
f = 0, 1
Ar est un groupe phényle ou pyridyle éventuellement substitué.
U étant un atome de chlore ou de brome
Y étant un atome de brome ou de chlore ou d'iode
B est $C_1$-$C_{10}$ alkyle, $C_3$-$C_{10}$ cycloalkyle, éventuellement substitués par 1 à 6 atomes d'halogène, phényle ou pyridyle ou pyridyle oxydé éventuellement substitués.
Utilisation de ces composés à titre d'herbicide sélectifs maïs notamment antigraminées de prélevée..

EP 0 398 818 A1

## Sulfones herbicides dihalogénées

L'invention est relative à de nouveaux composés, à leur utilisation à titre d'herbicides notamment sous forme de composition herbicide, à un procédé de contrôle des mauvaises herbes à l'aide de ces composés ou de ces compositions.

Un objet de la présente invention est donc de proposer des composés utiles en pré ou post émergence comme herbicides.

Un autre objet de la présente invention est de proposer des composés utiles en pré ou post émergence comme herbicides antigraminées.

Un autre objet de la présente invention est de proposer des composés utiles en pré ou post émergence comme herbicides sélectifs du maïs et de nombreuses cultures dicotylédones (notamment soja, colza, tournesol, coton) et autres monocotylédones (blé, riz).

<u>Définition générale de l'invention :</u>

Composés de formule :

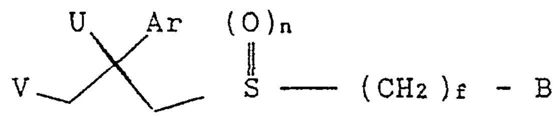

(I)

dans laquelle :

n = 0, 1, 2

f = 0, 1.

Ar est choisi parmi les groupes

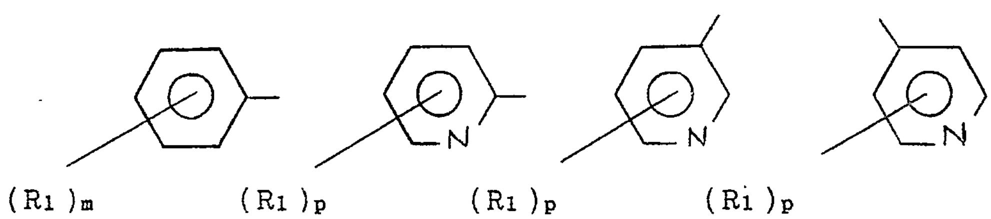

Ar-1 Ar-2 Ar-3 Ar-4

U étant un atome de brome ou de chlore,

V étant un atome de brome, de chlore ou d'iode,

$R_1$ étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou naphthoxy) éventuellement substitué par 1 ou 2 atomes d'halogène, $C_7$-$C_{11}$ aralkyloxy (notamment benzyloxy) éventuellement substitué par 1 ou 2 atomes d'halogène,

m = 0, 1, 2, 3, 4, 5,

p = 0, 1, 2, 3, 4,

les différents radicaux $R_1$ étant identiques ou différents lorsque m ou p est supérieur ou égal à 2,

B est choisi parmi les groupes $C_1$-$C_{10}$ alkyle, $C_3$-$C_{10}$ cycloalkyle, ces groupes étant éventuellement substitués par 1 à 6 atomes d'halogène ou choisis parmi les groupes

$B_1$ 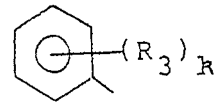 ; $B_2$ 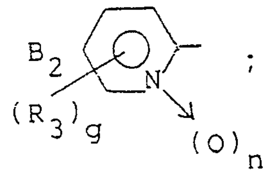 ; $B_3$ 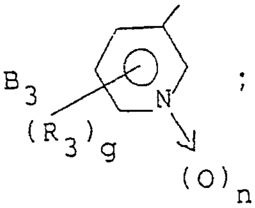 ; $B_4$ 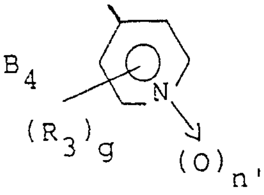

$R_3$ ayant l'une des significations indiquées pour $R_1$ ou $NR_4R_5$,$S(O)_hR_6$, $(C=O)R_7$,
$R_4$, $R_5$ identiques ou différents sont H, $C_1$-$C_4$ alkyle ou $C_6$-$C_{10}$ aryle, $R_6$ est $C_1$-$C_4$ alkyle,
$R_7$ est $C_1$-$C_4$ alkyle, $C_1$-$C_4$ alkoxy, $C_1$-$C_4$ haloalkoxy, $NR_9R_{10}$,
$R_9$, $R_{10}$ identiques ou différents sont H ou $C_1$-$C_4$ alkyle,
les différents radicaux $R_3$ étant identiques ou différents lorsque k ou g est supérieur ou égal à 2,
k = 0, 1, 2, 3, 4, 5,
g = 0, 1, 2, 3, 4,
h = 0, 1, 2,
n' = 0, 1.
Les groupes aliphatiques peuvent être linéaires ou ramifiés.

Variantes préférées :

Selon des modes de réalisation préférés, on choisira les variantes suivantes prises ou non en combinaison :
n= 2, U,V = Br, m inférieur ou égal à 3, p inférieur ou égal à 2, k inférieur ou égal à 2, g inférieur ou égal à 1, $R_1$ est halogène, nitro, trifluorométhyle, méthoxy, méthyle.

Les composés de formule (I) et les composés éventuelllement utilisables à titre d'intermédiaires dans les procédés de préparation, et qui seront définis à l'occasion de la description de ces procédés, peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

Procédés de préparation du composé de formule (I)

Les composés de formule (I) dans laquelle n = 0, 1, 2, les autres substituants ayant la même définition que celle indiquée dans la définition générale de l'invention peuvent être obtenus par mise en contact d'un composé de formule :

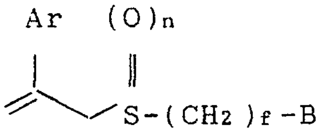 (IA)

dans laquelle Ar, n, f et B ont la même signification que dans la définition générale de l'invention, avec des halogénures UV comme le chlore (U = Cl, V = Cl), le brome (U = Br, Y = Br),l'iodure de chlore (U = Cl, V = I), l'iodure de brome (U = Br, V = I) ou le bromure de chlore (U = Cl, V = Br), dans un solvant inerte aprotique comme le chloroforme, le tétrachlorure de carbone, le tétrahydrofuranne, le diméthoxyéthane, l'acétonitrile en présence ou non d'un acide comme l'acide acétique ou l'acide chlorhydrique à une température de - 78 °C à 60 °C (de préférence 0 °C à 20 °C) et dans un rapport molaire IA : UV compris entre 1 et 5 (de préférence 1 et 2). Cette réaction est notamment connue par "J. March, Advanced Organic Chemistry" Ed. Mc Graw-Hill (1985), p. 724-726, S. Akiyoshi et K. Okuno J. Amer. Chem. Soc. (1952), 74, 5759 et F.G. Weber Tetrahedron (1969), 25, 4283.

Ces mêmes composés de formule (I) dans laquelle n = 0, 1 ou 2, les autres substituants ayant la

même définition que celle indiquée dans la définition générale de l'invention peuvent également être préparés par action sur un composé de formule (IA) ou les substituants ont la même signification que dans la définition générale de l'invention, d'un agent d'halogénation ZV, V étant l'atome de chlore ou de brome et Z un radical acétamido comme le N-halogéno acétamide, la N-halogéno succinimide en présence d'un donneur d'anion halogénure $U^-$ , U étant l'atome de chlore ou de brome, comme les acides halohydriques HU, les halogénures d'ammonium $(R)_4NU$, R étant un radical alkyle ou les sels MU, M étant un atome de métal alcalin, alcalino-terreux ou l'atome d'argent dans un solvant inerte comme le chlorure de méthylène, le chloroforme, l'acétonitrile, le diméthoxyéthane ou l'acide acétique à une température de - 78 ° C à 60 ° C (de préférence 0 ° C à 20 ° C) et dans un rapport molaire IA : ZV : $U^-$, compris entre 1 : 1 : 1 et 1 : 5 : 100 (de préférence 1 : 2 : 5). Cette réaction est notamment connue par J. March ibid. p. 725, R.E. Buckles et J.W. Long J. Amer. Chem. Soc. (1959), 81, 2191, A. Marquet et J. Jacques Tetrahedron Letters (1959), 9, 24 et C.H. Robinson et al. J. Amer. Chem. Soc (1959), 81, 2191.

Les composés de formule (I) dans laquelle n = 0 ou 1, les autres substituants ayant la même définition que celle indiquée dans la définition générale de l'invention, peuvent être oxydés en sulfoxyde (n = 1) par un équivalent d'oxydant à un température de - 70 ° C à 5 ° C (généralement 0 ° C) ou être oxydés en sulfone (n = 2) par deux ou plus équivalents d'oxydant à une température de 0 ° C à 60 ° C (généralement 10 ° C à 30 ° C) par de nombreux oxydants comme $KMnO_4$, $H_2O_2$, $CH_3CO_3H$, acides perben-zoïques, $KHSO_5$ et d'autres suivant de très nombreuses méthodes connues J. March ibid. p. 1089-1090 et B.M. Trost et R. Braslau J. Org Chem. (1988), 53, 532.

Procédé de préparation du composé de formule IA

Méthode A

Les composés de formule (IA) pour lesquels n = 2, les autres substituants ayant la même définition que celle indiquée dans la définition générale de l'invention peuvent être obtenus par mise en contact d'un composé de formule :

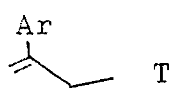

(II)

dans laquelle Ar, $R_1$ et m ou p ont la même signification que dans la définition générale de l'invention,
T est un atome de chlore ou de brome, avec un composé de formule
$MSO_2\ (CH_2)_f$ - B (III)
f, B ayant la même définition que celle indiquée dans la définition de l'invention,
M étant un atome de métal alcalin ou alcalino-terreux (notamment Li, K, Na).

La réaction est généralement effectuée dans un solvant aprotique dipolaire notamment le diméthylformamide, la N-méthyl pyrrolidone, ou dans l'eau en mélange dans les proportions 5/95 à 90/10 (de préférence 10/90 à 50/50) avec un solvant soluble dans l'eau comme les alcools, l'acétone, l'acétonitrile le diméthoxyéthane, en présence ou non, d'une quantité catalytique ou non d'iodure alcalin, en présence ou non, d'une quantité catalytique d'un agent de transfert de phase comme les halogènures de tétrabutylammonium, à une température comprise entre 25 ° C et 150 ° C (de préférence 60 ° C à 120 ° C) et dans un rapport molaire II:III compris entre 1 et 10 (de préférence 1 et 2).

Cette réaction est notamment connue par J. March ibid. p. 363. Les composés de formule (II) où T est chlore, et Ar est Ar-1 (noyau phényle) sont préparés par chloration d'un composé 2-phényl 1-propène de formule:

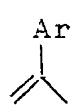

(IV)

dans laquelle Ar est Ar-1 (noyau phényle),
$R_1$ et m ayant la même signification que dans la définition de la formule générale, au moyen du réactif Ca-

$(OCl)_2/CO_2$. Cette réaction est décrite par S.G. Hegde et J. Wolinsky Tetrahedron Letters (1981), 22, 5019.

Ces mêmes composés de formule (II) où T est chlore, et Ar est Ar-1 (noyau phényle), peuvent être préparés par chloration des composés de formule (IV) au moyen du réactif $SO_2Cl_2/Na_2CO_3$ selon M. Bulliard et al. Tetrahedron Letters (1989), 30, 5767.

Les composés de formule (II) où T est chlore, Ar ayant la même définition que celle indiquée dans la définition générale de l'invention, peuvent également être préparés par chloration des composés de formule (IV) sus indiqués au moyen de N-chloro succinimide en présence de bisaryldisélénide suivant le procédé de K.B. Sharpless et T. Hori J. Org. Chem. (1979), 44, 4204.

Les composés de formule (II) où T est chlore ou brome, Ar ayant la même définition que celle indiquée dans la définition générale de l'invention, peuvent également être préparés par halogénation radicalaire thermique ou photochimique par la N-halogéno succinimide du composé de formule (IV), dans un solvant aprotique comme le tétrachlorure de carbone ou en l'absence de solvant, avec ou non un initiateur de radicaux libres, à une température de 20° C à 170° C (de préférence 80° C à 100° C) suivant S.F. Reed J. Org. Chem. (1965), 30, 3258. Ils peuvent encore être préparés par halogénation des composés de formule (II) où T est OH, Ar ayant la même définition que celle indiquée dans la définition générale de l'invention, avec un agent d'halogénation tel que $SOCl_2$, $POCl_3$, $PBr_3$, J. March ibid. p. 382-384 ou avec le réactif $LiCl/CH_3SO_2Cl$/collidine suivant E.W. Collington et A.I. Meyers J. Org. Chem (1971), 36, 3044.

Les composés de formule (II) où T est OH, Ar ayant la même définition que celle indiquée dans la définition générale de l'invention, peuvent être préparés par oxydation allylique du composé de formule (IV) par l'oxyde de sélénium catalytique ou non, en présence d'un oxydant comme l'hydroperoxyde de tertiobutyle dans un solvant inerte comme les solvants halogénés (de préfé-rence le chlorure de méthylène ) ou le tertiobutanol, en présence d'acide minéral ou organique selon M.A. Umbreit et K.B. Sharpless J. Amer. Chem. Soc. (1977), 99, 5526.

Les composés de formule (IV) peuvent être obtenus par déshydratation d'un composé 2-aryl 2-propanol de formule :

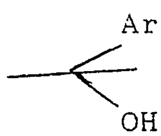

(V)

dans laquelle Ar, $R_1$ et m ou p ont la même signification que dans la définition générale de l'invention, par des agents de déshydratation comme $P_2O_5$, $KHSO_4$, $POCl_3$/py-ridine et d'autres selon J. March ibid. p. 901-903.

Les composés de formule (V) peuvent être préparés par mise en contact de l'acétophénone ou acétylpyridine ou dérivé d'acide de formule :

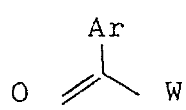

(VI)

dans laquelle Ar, $R_1$ et m ou p ont la même signification que dans la définition générale de l'invention et W est méthyle, alkoxy (ester benzoïque correspondant) ou chlore avec un ou deux équivalents d'halogénure de méthylmagnésium suivant J. March ibid. p. 816-822.

Les composés de formule (VI) sont obtenus de manière connue en soi. Les composés de formule (III) peuvent être préparés par réduction des halogénures de sulfonyle correspondants (généralement chlorure) par le zinc, l'iodure de sodium ou de potassium, le sulfite de sodium selon J. March ibid. p. 445-446, et W.E. Truce et A.H. Murphy Chem. Rev. (1951), 48, 69. Les halogénures de sulfonyle peuvent être préparés selon J. March ibid p. 1172.

Les composés de formule (III) peuvent être également préparés par action d'un organométallique (habituellement lithien) de formule :

$M\ (CH_2)_f - B$ (IIIA)

f, B ayant la même signification que celle indiquée dans la définition de l'invention, M étant notamment lithium, avec l'anhydride sulfureux $SO_2$ , à une température comprise entre -78° C et 20° C (de préférence -78° C à -40° C) en l'absence ou en présence d'un solvant aprotique comme l'éther éthylique ou le

tétrahydrofuranne selon H.W. Pinnick et M.A. Reynolds J. Org. Chem. (1979), 44, 160, et J. March ibid. p. 550.

Les composés de formule (IIIA) sont obtenus de manière connue en soi.

Méthode B

Les composés de formule (IA) dans laquelle n = 0, 1 ou 2 peuvent être préparés par action du sel alcalin d'un thiolate d'aryle ou d'alkyle de formule :

M′ S $(CH_2)_f$ - B (VII)

dans laquelle M′ est un atome de métal alcalin ou alcalinoterreux notamment sodium ou potassium, f et B ayant la même définition que celle indiquée dans la définition de l'invention sur un composé de formule (II) où T est halogène précédemment décrit, dans un solvant inerte protique ou aprotique tel que les cétones, les alcools, le tétrahydrofuranne, l'acétonitrile, les solvants dipolaires aprotiques comme le diméthylformamide, à une température de 0° C à 80° C (généralement 25° C a 60° C) dans un rapport molaire II:VII compris généralement entre 1 et 10 (de préférence 1 et 2).

Le sulfure ainsi obtenu (n = 0) peut être oxydé en sulfoxyde (n = 1) par un équivalent d'oxydant à une température de - 70° C à 5° C (généralement 0° C) ou être oxydé en sulfone (n = 2) par deux ou plus équivalents d'oxydant à une température de 0° C à 60° C (généralement 10° C à 30° C) par de nombreux oxydants comme $KMnO_4$, $H_2O_2$, $CH_3CO_3H$, acides perbenzoïques, $KHSO_5$ et d'autres suivant de très nombreuses méthodes connues : J. March ibid. p. 1089-1090, et B.M. Trost et R. Braslau J. Org. Chem. (1988), 53, 532.

L'invention a également pour objet les produits (II) à (VII) nouveaux utiles pour la mise en oeuvre du procédé qui vient d'être décrit

Les exemples suivants illustrent l'invention :

Exemple 1

On dissout 10,3 g (0,04 mole) de 2-phényl 1-phénylsulfonyl 2-propène dans 100 $cm^3$ de chloroforme. On ajoute alors goutte-à-goutte 6,8 g (0,042 mole) de brome jusqu'à coloration persistante. La phase organique est lavée par 50 $cm^3$ d'une solution à 5 % de thiosulfate de sodium, 50 $cm^3$ d'eau et séchée sur $MgSO_4$. Après évaporation, on obtient 18,8 g d'une huile incolore. Cette huile est dissoute dans un mélange de 20 $cm^3$ de chloroforme et 40 $cm^3$ d'éther et stockée à -18° C pendant 1 nuit.On filtre 14,3 g (86 %) de cristaux blancs de 1,2-dibromo 2-phényl 3-phénylsulfonylpropane. F = 74° C.

Exemple 2

On dissout 2,6 g (0,01 mole) de 2-phényl 1-phénylsulfonyl 2-propène dans 20 $cm^3$ de chloroforme On re-froidit à 5° C et ajoute goutte-à-goutte 1,8 g (0,011 mole) d'iodure de chlore en solution dans 5 $cm^3$ de chloroforme Le milieu est agité deux heures à 5° C puis traité d'une manière identique à l'exemple 1. L'évaporation laisse 5,3 g d'une huile jaune. Cette huile est dissoute dans 7,5 $cm^3$ d'éther et stockée à -18° C pendant 1 nuit. On filtre 3,3 g (78 %) de cristaux blanc cassé de 2-chloro 1-iodo 2-phényl 3-phénylsulfonylpropane. F = 75,5° C (dec.).

Exemple 3

On met en suspension 2,6 g (0,01 mole) de 2-phényl 1-phénylsulfonyl 2-propène dans un mélange de 50 $cm^3$ d'acide acétique et 10 $cm^3$ d'acide chlorhydrique à 35 % Du chlore est alors introduit dans le milieu réactionnel jusqu'à dissolution totale de la sulfone allylique (5mn). Le milieu est versé dans 250 g de glace et d'eau et est fortement agité jusqu'à formation d'une gomme.

La gomme est filtrée, dissoute dans 100 $cm^3$ de chlorure de méthylène et la phase organique est lavée par 100 $cm^3$ d'une solution à 10 % de carbonate de sodium, 2 x 100 $cm^3$ d'eau et séchée sur $MgSO_4$. L'évaporation laisse 4,8 g d'une huile jaune. Cette huile est dissoute dans 20 $cm^3$ d'éther et stockée à -18° C pendant 1 nuit. Après filtration et recristallisation dans l'éthanol on obtient 1,2 g (36 %) de cristaux jaune clair de 1,2-dichloro 2-phényl 3-phénylsulfonylpropane. F = 95° C.

Exemple 4

On met en suspension 2,6 g (0,01 mole) de 2-phényl 1-phénylsulfonyl 2-propène dans 50 $cm^3$ d'acide acétique. On ajoute alors 2 g (0,05 mole) de chlorure de lithium, 5 $cm^3$ d'acide chlorhydrique à 35 % et enfin 1,8 g (0,01 mole) de N-bromosuccinimide. Le milieu est agité jusqu'à dissolution (15 mn) puis traité d'une manière identique à l'exemple 3. L'évaporation laisse 3,6 g d'une huile jaune qui est redissoute dans 10 $cm^3$ d'éther et stockée à -18° C pendant 1 nuit. On filtre 1,5 g (40 %) de cristaux blancs de 1-bro-mo 2-chloro 2-phényl 3-phénylsulfonyl propane. F = 91° C.

Exemple 5

D'une manière identique à l'exemple 1, on traite 1,0 g (0,0035 mole) de 2-(6-chloro 2-pyridyl) 1-phénylsul-fonyl 2-propène par 0,6 g (0,0038 mole) de brome dans le chloroforme. Après traitement, l'évaporation laisse 1,6 g d'une huile qui est cristallisée dans 40 $cm^3$ d'éther à -18° C pendant 1 nuit. On filtre 1,2 g (75 %) de cristaux blancs de 1,2-dibromo 2-(6-chloro 2-pyridyl) 3-phénylsulfonylpropane. F = 119° C.

Exemple 6

D'une manière identique à l'exemple 1, on traite 1,4 g (0,0054 mole) de 2-(6-chloro 2-pyridyl) 1-cyclopropylsulfonyl 2-propène par 0,87 g (0,0054 mole) de brome dans le chloroforme. Après traitement, l'évaporation laisse 2,1 g d'une huile qui est chromatographiée sur silice (éluant : heptane/chloroforme 80/20) pour donner 0,7 g (31 %) de cristaux de 1,2-dibromo 2-(6-chloro 2-pyridyl) 3-cyclopropylsulfonylpropane. F = 75° C. Les composés rassemblés dans le tableau ci-après ont été préparés selon la méthode de l'exemple 1

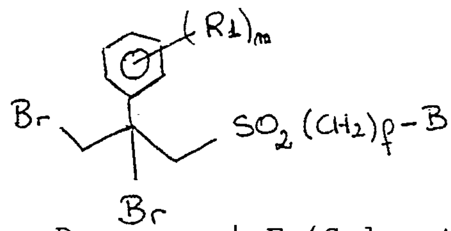

| Exemple | R1 | f | B | F (Solvant) Nᴅ (température) |
|---|---|---|---|---|
| 7 | 2-F | 0 | phényl | 123°C ($CHCl_3$/Pentane) |
| 8 | 4-Me | 0 | phényl | 101°C ($CHCl_3$/$Et_2O$) |
| 9 | 4-F | 0 | phényl | 103°C ($CHCl_3$/$Et_2O$) |
| 10 | 3,5-diCl | 0 | phényl | 135°C ($CHCl_3$/$Et_2O$) |
| 11 | H | 1 | phényl | 93°C ($CHCl_3$/$Et_2O$) |
| 12 | H | 0 | 4-F phényl | 103°C ($CHCl_3$/Pentane) |
| 13 | H | 0 | 2-Cl phényl | 120°C ($CHCl_3$/Pentane) |
| 14 | H | 0 | 2-F phényl | 82°C ($Et_2O$) |
| 15 | H | 0 | 2-Me phényl | 74°C ($CHCl_3$/$Et_2O$) |
| 16 | H | 0 | 3-Me phényl | 95°C ($CHCl_3$/$Et_2O$) |
| 17 | H | 0 | 2-Cl 4-F phényl | 110°C $Et_2O$/Pentane) |
| 18 | 3-Cl | 0 | 2-Cl phényl | 87°C ($CH_2Cl_2$/$iPr_2O$) |
| 19 | 3-F | 0 | 2-Cl phényl | 97°C ($Et_2O$/Pentane) |
| 20 | 3-$CF_3$ | 0 | 2-Me phényl | 92°C ($Et_2O$) |
| 21 | 3,5-diCl | 0 | 4-F phényl | 133,5°C ($CHCl_3$/$Et_2O$) |
| 22 | 3,5-diCl | 0 | 2-Me phényl | 117°C (chromatographie) |
| 23 | H | 0 | $(CH_2)_3CH_3$ | 1,5768 (25°C) |
| 24 | H | 0 | Cyclopropyl | 95°C ($Et_2O$) |
| 25 | 3-Cl | 0 | $(CH)CH_3)_2$ | 1,5717 (24°C) |

Préparation du 2-phényl 1-phénylsulfonyl 2-propène

On dissout 130 cm³ (1 mole) de 2-phényl 1-propène dans 1 500 cm³ de chlorure de méthylène. On ajoute 300 cm³ d'eau et 101,5 g (0,5 mole) d'hypochlorite de calcium à 70 % en chlore actif. Sous très forte agitation, on additionne pendant 2 heures de la carboglace. On décante les deux phases et sèche la phase organique sur $MgSO_4$.

Après évaporation, on obtient 145 g d'une huile jaune.

L'analyse RMN (60 MHz) révèle la présence de 45 % de 1-chloro 2-phényl 2-propène, 45 % de 1-chloro 2-phényl 1-propène et 10 % de 1-chloro 2-phényl 2-propanol.

17 g de ce mélange (0,05 mole en 1-chloro 2-phényl 2-propène) sont dissous dans 100 cm³ de diméthylfo-ramide. On ajoute 8,2 g (0,05 mole) de benzènesulfinate de sodium et chauffe 2 h à 70° C. Le mélange réactionnel est versé dans 350 g de glace et d'eau On agite for-tement avec 150 cm³ de pentane pour extraire le 1-chloro 2-phényl 1-propène et le 1-chloro 2-phényl 2-propanol qui n'ont pas réagi, pendant 15 à 30 mn jusqu'à cristallisation complète. On filtre les cristaux formés, lave par 50 cm³ de pentane, 50

$cm^3$ de diisopropyléther, essore à fond et sèche. On obtient 9,8 g (76 %) de 2-phenyl 1-phénylsulfonyl 2-propène. F = 105° C.

De la même manière ont été préparés les composés :
2-(2-fluorophényl) 1-phénylsûlfonyl 2-propène, F= 95° C,
2-(4-méthylphényl) 1-phénylsulfonyl 2-propène, F= 92° C,
2-(4-fluorophényl) 1-phénylsulfonyl 2-propène, F= 85° C,
2-(3-chlorophényl) 1-(2-chlorophénylsulfonyl 2-propène, F = 87° C,
1-(2-chlorophénylsulfonyl)2-phényl2-propène $n^{22}$ = 1,6090,
2-(3,5-dichlorophényl) 1-phénylsulfonyl 2-propène,F=98° C,
1-(2-chlorophénylsulfonyl) 2-(3-fluorophényl) 2-propène, $n_D^{24}$ = 1,5860,
1-(2-fluorophénylsulfonyl) 2-phényl 2-propène, F= 84° C,
1-butylsulfonyl 2-phényl 2-propène, F = 25° C,
1-cyclopropylsulfonyl 2-phényl 2-propène, F = 76° C.

Préparation du 1-benzylsulfonyl 2-phényl 2-propène

On met en suspension 27 g (0,19 mole) de carbonate de potassium dans 600 $cm^3$ d'acétone. Le milieu est chauffé à 60° C et est dégazé à l'azote. On ajoute goutte-à-goutte à 60° C un mélange de 21,7 g (0,17 mole) de benzylmercaptan et 59 g (0,17 mole en 1-chloro 2-phényl 2-propène) du chlorure allylique préparé selon l'exemple précédent en solution dans 175 $cm^3$ d'acétone. Le milieu est agité deux heures à 60° C.

Le mélange réactionnel est versé dans 2 000 g de glace et d'eau, réextrait par 3 x 500 $cm^3$ d'éther. La phase organique est lavée à l'eau jusqu'à neutralité et séchée sur $MgSO_4$. L'évaporation laisse 82,5 g d'une huile jaune qui est chromatographiée sur silice (éluant : heptane puis heptane/chloroforme 99/l) pour donner 19,5 g (46 %) de 1-benzylthio 2-phényl 2-propène. Huile incolore. $n_D^{25}$ = 1,6041.

13,2 g (0,055 mole) de 1-benzylthio 2-phényl 2-propène sont dissous dans 300 $cm^3$ de méthanol et 300 $cm^3$ d'eau. On ajoute par portions 36,9 g (0,12 mole en $KHSO_5$) d'Oxone et agite trois heures à température ambiante. On dilue le mélange réactionnel par 1 000 $cm^3$ d'eau, ajoute 100 $cm^3$ de pentane et agite fortement jusqu'à cristallisation. 10,7 g (72 %) de cristaux blancs de 1-benzylsulfonyl 2-phényl 2-propène sont obtenus par filtration puis séchage. F = 118° C.

De la même manière ont été préparés les composés :
2-(3-chlorophényl) 1-isopropylsulfonyl 2-propène, $n_D^{25}$ = 1,5715,
1-(4-fluorophénylsulfonyl) 2-phényl 2-propène, F= 83° C,
1-(3-méthylphénylsulfonyl) 2-phényl 2-propène, F= 61° C,
1-(2-méthylphénylsulfonyl) 2-phényl 2-propène, F= 62° C,
1-(2-chloro 4-fluorophénylsulfonyl) 2-phényl 2-propène, F = 64° C,
2-(3,5-dichlorophényl) 1-(4-fluorophénylsulfonyl) 2-propène, F = 105° C,
2-(3,5-dichlorophényl) 1-(2-méthylphénylsulfonyl 2-propène, F = 72° C,
1-(2-méthylphénylsulfonyl) 2-(3-trifluorométhylphényl) 2-propène, F = 51° C.

Préparation du 2-(6-chloro 2-pyridyl) 1-phénylsulfonyl 2-propène

On dissout 15,3 g (0,1 mole) 2-(6-chloro 2-pyridyl) 1-propène dans 200 $cm^3$ de 1,2-dichloroéthane. On ajoute 0,5 g (catalytique) de bis(4-chlorophényl) disélénide et 14,7 g (0,11 mole) de N-chlorosuccinimide et chauffe 24 heures à 60° C. On concentre le milieu réactionnel au tiers, filtre le succinimide, et lave la phase organique par 2 x 200 $cm^3$ d'eau, 1 x 200 $cm^3$ d'une solution à 15 % de bicarbonate de sodium, 2 x 200 $cm^3$ d'eau et séche sur $MgSO_4$. Brut : 13,9 g.

L'analyse RMN (60 MHz) révèle la présence de 55 % de 1-chloro 2-(6-chloro 2-pyridyl) 2-propène et 45 % de 1-chloro 2-(6-chloro 2-pyridyl) 1-propène.

D'une manière identique à la préparation du 2-phényl 1-phénylsulfonyl 2-propène, on traite 7 g de ce mélange (0,02 mole en 1-chloro 2-(6-chloro 2-pyridyl) 2-propène) par le benzène sulfinate de sodium pour obtenir après traitement 4,3 g (73 %) de 2-(6-chloro 2-pyridyl) 1-phénylsulfonyl 2-propène. F = 135° C.

De la même manière a été préparé le composé :
2-(6-chloro 2-pyridyl) 1-cyclopropylsulfonyl 2-propène N $_D^{24}$ = 1,5730.

L'invention concerne également l'utilisation à titre d'herbicide des composés de formule (I). Comme mauvaisesherbes pouvant être contrôlées ou détruites par les composés de formule (I), on peut citer :

| Graminées / Cypéracées | | |
|---|---|---|
| Abreviation | Nom latin | Nom français |
| AVE | Avena fatua | Folle avoine |
| ECH | Echinochloa crusgalli | Panisse |
| LOL | Lolium multiflorum | Ray-grass |
| CYP | Cyperus esculentus | Cyperus |
| DIG | Digitaria sanguinalis | Digitaire |
| ALO | Alopecurus myosuroedes | Vulpin |

L'utilisation des composés de formule (I) est la plupart du temps sous forme de composition herbicide comportant un ou plusieurs supports acceptables en agriculture.

En effet pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en mélange avec les supports solides ou liquides, acceptables en agriculture et les agents tensio-actifs également acceptables en agriculture.En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... . Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc..).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents.

La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de $5.10^{-5}$% à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

| Exemple F 1 : | |
|---|---|
| - matière active (composé n° 1) ..... | 50 % |
| - alcool gras éthoxylé (agent mouillant)..... | 2,5 % |
| - phényléthylphénol éthoxylé (agent dispersant).. | 5 % |
| - craie (support inerte) ..... | 42,5 % |

| Exemple F 2 : | |
|---|---|
| - matière active (composé n° 1) ..... | 10% |
| - alcool synthétique oxo de type ramifié, en $C_{13}$ éthoxylé par 8 à 10 oxyde d'éthylène (agent mouillant) ..... | 0,75 % |
| - lignosulfonate de calcium neutre (agent dispersant ..... | 12 % |
| - carbonate de calcium (charge inerte) ....q.s.p. | 100 % |

Exemple F 3 :

cette poudre mouillable contient les mêmes ingrédients que dans l'exemple précédent, dans les proportions ci-après :

| | |
|---|---|
| - matière active ..... | 75 % |
| - agent mouillant ..... | 1,50 % |
| - agent dispersant ..... | 8 % |
| - carbonate de calcium (charge inerte) ....q.s.p. | 100 % |

| Exemple F 4 : | |
|---|---|
| - matière active (composé n° 1) ..... | 90 % |
| - alcool gras éthoxylé (agent mouillant ..... | 4 % |
| - phényléthylphénol éthoxylé (agent dispersant) ... | 6 % |

| Exemple F 5 : | |
|---|---|
| - matière active (composé n° 1) ..... | 50 % |
| - mélange de tensio-actifs anioniques et non ioniques (agent mouillant) ..... | 2,5 % |
| - lignosulfonate de sodium (agent dispersant) .... | 5 % |
| - argile kaolinique (support inerte) ..... | 42,5 % |

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple F 6 : Granulés dispersibles

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F 7 : Granulés dispersibles

Dans un mélangeur, on mélange les constituants suivants :

| | |
|---|---|
| - matière active (composé n° 1) ..... | 75 % |
| - agent mouillant (alkylnaphtalène sulfonate de sodium ..... | 2 % |
| - agent dispersant (polynaphtalène sulfonate de sodium) ..... | 8 % |
| - charge inerte insoluble dans l'eau (kaolin) ..... | 15 % |

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir

la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

| Exemple F 8 : | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique q.s.p | 1 litre |

Selon une autre formule de concentré émulsionnable, on utilise :

| Exemple F 9 : | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

| Exemple F 10: | |
|---|---|
| - composé | 500 g |
| - phosphate de tristyrylphénol polyéthoxylé | 50 g |
| - alkylphénol polyéthoxylé | 50 g |
| - polycarboxylate de sodium | 20 g |
| - éthylène glycol | 50 g |
| - huile organopolysiloxanique (antimousse) | 1 g |
| - polysaccharide | 0,5 g |
| - eau | 316,5 g |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans -l'eau et elles peuvent avoir une consistance épaisse comme celle d'une "mayonnaise".

En ce qui concerne les compositions adaptées au stockage et au transport, elles contiennent plus avantageusement de 0,5 à 95 % (en poids) de substance active.

La présente invention concerne aussi un procédé de désherbage (notamment de zones de culture (monocotylédones (blé mais, riz) qui consiste à appliquer aux plantes devant être détruites une quantité efficace d'un composé de formule (I).

Lors de l'application à une zone cultivée, la dose d'application devrait être suffisante pour contrôler la naissance des adventices sans causer de dommages substantiels permanents auxdites cultures. Par dose efficace, on entend justement dans ce contexte, la dose qui permet d'obtenir ce résultat.

Les produits et compositions selon l'invention s'appliquent de préférence sur des zones ou terrains où l'on veut empêcher la pousse ou le développement de plantes n'ayant pas encore poussé (application de préémergence). Neanmoins, on pourra utiliser également un procédé de désherbage qui consiste à appliquer une quantité efficace d'un composé de formule (I) sur les mauvaises herbes à éliminer lorsque celles-ci présentent un feuillage vert, avantageusement les monocotylédones.

On peut opérer de manière à ce que la culture soit semée avant ou après le traitement.

La dose d'application de matière active est généralement comprise entre 1 et 8 000 g/ha.

Les exemples ci-desous illustrent l'invention :

Exemple A - Application herbicide, en prélevée des espèces végétales

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une solution ou suspension de la matière active dans un mélange acétone/eau en proportions 50/50, en présence de 0,05 % en poids de Cemulsol NP 10 (agent tensio-actif) constitue d'alkylphénol polyéthoxylé, notamment de nonylphénol polyéthoxylé) et 0,04 % en poids de tween 20 (agent tensio-actif constitué d'un oléate de dérivé polyoxyéthyléné du sorbitol).

Dans le cas d'une suspension, celle-ci est obtenue en mélangeant et broyant les ingrédients dans un microniseur de façon à obtenir une grosseur moyenne de particules inférieure à 40 microns.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) des destructions du nombre de pieds dans le pot traité

par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| | Notation |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Lés résultats obtenus sont présentés après l'exemple B pour des doses d'application de 4000 g/ha.

Exemple B - Application herbicide, en postlevée des espèces végétales

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation ". Le stade de traitement pour les dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouille a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| | Notation |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Les résultats obtenus sont présentés après le tableau A pour des doses d'application de 4000 g/ha.
Les espèces végétales utilisées dans ces exemples A et B sont :

| ABREVIATIONS | NOM LATIN | NOM FRANCAIS |
|---|---|---|
| AVE | Avena fatua | Folle avoine |
| ALO | Alopecurus myosuroïdes | Vulpin |
| ECH | Echinochloa crusgalli | Panisse |
| CYP | Cyperus esculentus | Cyperus |
| DIG | Digitaria sanguinalis | Digitaire |

| ACTIVITE HERBICIDE DE PRELEVEE | | | | | |
|---|---|---|---|---|---|
| COMPOSES N° | AVE | ECH | DIG | CYP | ALO |
| 1 | 5 | 5 | 5 | 3 | - |
| 2 | 1 | 4 | 4 | 0 | - |
| 4 | 5 | 5 | 5 | 3 | 5 |
| 8 | 2 | 4 | 5 | 1 | 2 |
| 9 | 5 | 5 | 5 | 3 | 4 |
| 11 | 1 | 5 | 5 | 3 | 5 |
| 12 | 5 | 5 | 5 | 3 | 5 |
| 13 | 5 | 5 | 5 | 2 | 5 |
| 14 | 4 | 5 | 5 | 4 | 5 |
| 15 | 5 | 5 | 5 | 3 | 5 |
| 16 | 3 | 5 | 5 | 3 | 5 |
| 17 | 3 | 5 | 5 | 3 | 5 |
| 23 | 0 | 5 | 5 | 1 | 5 |
| 24 | 5 | 5 | 5 | 2 | 5 |
| 25 | 5 | 5 | 5 | 3 | 5 |

| ACTIVITE HERBICIDE DE POSTLEVEE | | | |
|---|---|---|---|
| COMPOSES N° | ECH | DIG | ALO |
| 11 | 0 | 3 | 3 |
| 13 | 4 | 3 | 2 |
| 15 | 5 | 3 | 3 |
| 16 | 3 | 0 | 3 |
| 17 | 3 | 0 | 3 |
| 24 | 3 | 1 | 3 |

Exemple C - Essai de sélectivité en grandes cultures avec application herbicide, en prélevée des espèces végétales

Dans des pots de 7 X 7 X 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :
Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoins.

Le pourcentage de volume foliaire non détruit par le produit est donc donné par la formule :

$$\frac{[100-D] \times [100-RT]}{100} = A$$

Cette valeur A est transformée en notation de 0 à 5 selon l'échelle suivante :

| | notation |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Ainsi, un produit est jugé comme sélectif vis à vis de la culture lorsque la valeur A notée est de 0 ou 1.

Les résultats obtenus sont présentés dans l'exemple C pour des doses d'application de 1 ou 2 ou 4 kg de matière active par hectare selon les produits.

Les espèces végétales utilisées dans cet exemple sont :

| 1) Pour les adventices | | |
|---|---|---|
| ABREVIATIONS | NOM LATIN | NOM FRANCAIS |
| ECH | Echinochloa crus-galli | Panisse |
| DIG | Digitaria sanguinalis | Digitaire |
| SOR | Sorghum halepense | Sorgho d'Alep |
| SET | Setaria faberii | Millet |

| 2) Pour les cultures | | |
|---|---|---|
| ABREVIATIONS | NOM LATIN | NOM FRANCAIS |
| TRZ | Triticum aestivum | Blé |
| ZEA | Zea mays | Maïs |
| ORY | Oryza sativa | Riz |
| GLX | Glycine maximum | Soja |

| COMPOSES N° | DOSE APPLIQUEE (Kg/ha) | ESSAI DE SELECTIVITE EN GRANDES CULTURES ACTIVITE HERBICIDE DE PRELEVEE | | | | | | | |
|---|---|---|---|---|---|---|---|---|---|
| | | ECH | DIG | SOR | SET | TRZ | ZEA | ORY | GLY |
| 1 | 2 | 3 | 5 | 5 | 2 | 0 | 0 | 0 | 1 |
| 4 | 2 | 5 | 5 | 1 | 5 | 0 | 0 | 1 | 0 |
| 9 | 2 | 5 | 5 | 2 | 2 | 0 | 0 | 3 | 0 |
| 13 | 2 | 4 | 5 | 2 | 4 | 0 | 1 | 2 | 0 |
| 14 | 2 | 3 | 5 | 3 | 4 | 0 | 2 | 0 | 0 |
| 15 | 2 | 5 | 5 | 5 | 5 | 1 | 0 | 3 | 0 |

Comme on peut le voir sur le tableau de résultats de cet exemple C, de nombreux produits présentent une excellente activité antigraminée de prélevée tout en montrant une excellente sélectivité pour 1 ou 2 ou 3 ou 4 des 4 cultures testées = blé, maïs, riz, soja.

## Revendications

1) Composés de formule :

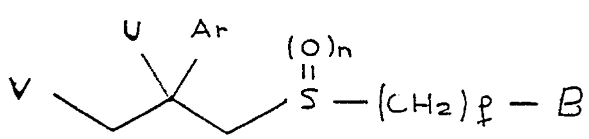

I

dans laquelle :
n = 0, 1, 2
f = 0, 1
Ar est choisi parmi les groupes

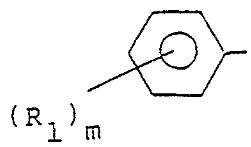
Ar-1

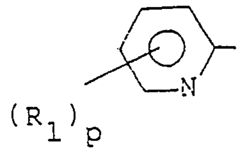
Ar-2

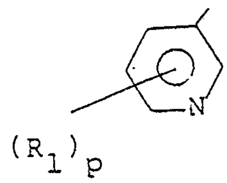
Ar-3

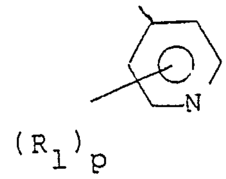
Ar-4

U étant un atome de brome ou de chlore,
V étant un atome de chlore, de brome ou d'iode,
$R_1$étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou napthoxy) éventuellement substitué par 1 ou 2 atomes d'halogène, $C_7$-$C_{11}$ aralkyloxy (notamment benzyloxy) éventuellement substitué par 1 ou 2 atomes d'halogène,
m = 0, 1, 2, 3, 4, 5
p = 0, 1, 2, 3, 4
les différents radicaux $R_1$ étant identiques ou différents lorsque m ou p est supérieur ou égal à 2.
B est choisi parmi les groupes $C_1$-$C_{10}$ alkyle, $C_3$-$C_{10}$ cycloalkyle, ces groupes étant éventuellement substitués par 1 à 6 atomes d'halogène ou choisi parmi les groupes

$B_1$ 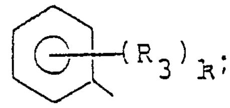 ; $B_2$ 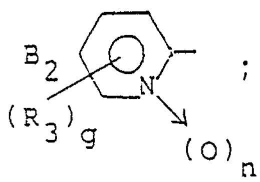 ; $B_3$ 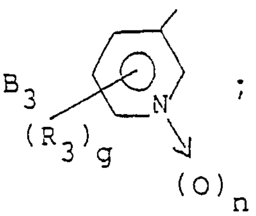 ; $B_4$ 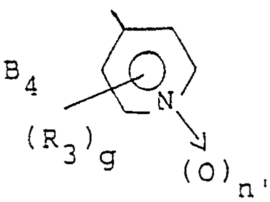

$R_3$ ayant l'une des significations indiquées pour $R_1$ ou $NR_4R_5$,$S(O)_hR_6$, $(C=O)R_7$,
$R_4$, $R_5$ identiques ou différents sont H, $C_1$-$C_4$ alkyle ou $C_6$-$C_{10}$ aryle, $R_6$ est $C_1$-$C_4$ alkyle,
$R_7$ est $C_1$-$C_4$ alkyle, $C_1$-$C_4$ alkoxy, $C_1$-$C_4$ haloalkoxy, $NR_9R_{10}$,
$R_9$, $R_{10}$ identiques ou différents sont H ou $C_1$-$C_4$ alkyle, les différents radicaux $R_3$ étant identiques ou différents lorsque k ou g est supérieur ou égal à 2,
k = 0, 1, 2, 3, 4, 5,
g = 0, 1, 2, 3, 4,
h = 0, 1, 2,
n' = 0, 1.

2) Composé selon la revendication 1, caractérisé en ce que n = 2.

3) Composé selon la revendication 1, caractérisé en ce que U,V correspondent à l'atome de brome.

4) Composé selon la revendication 1, caractérisé en ce que m inférieur ou égal à 3.

5) Composé selon la revendication 1, caractérisé en ce que p inférieur ou égal à 2.

6) Composé selon la revendication 1, caractérisé en ce que k inférieur ou égal à 2.

7) Composé selon la revendication 1, caractérisé en ce que g inférieur ou égal à 1.

8) Composé selon la revendication 1, caractérisé en ce que $R_1$ est halogène, nitro, trifluorométhyle, méthoxy, méthyle.

9) Utilisation des composés selon l'une des revendications 1 à 8 à titre d'herbicide notamment sous la forme d'une composition herbicide comportant en outre un support inerte.

10) Composition herbicide comportant 0,05 à 95 % en poids d'une matière active selon l'une des revendications 1 à 9 en combinaison avec les supports solides ou liquides acceptables en agriculture et les agents tensio actif acceptables en agriculture.

11) Procédé de préparation des composés selon l'une des revendications 1 à 9 caractérise en ce que ils sont préparés par mise en contact d'un composé de formule IA

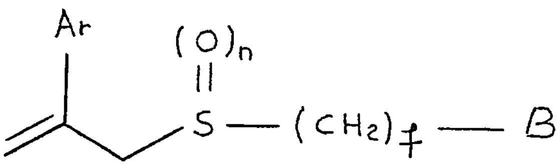

dans laquelle Ar, n, f et B ont la même signification que dans la revendication 1 et d'un halogènure XY, X, Y ayant la même signification que dans la revendication 1.

Office européen des brevets

# RAPPORT DE RECHERCHE EUROPEENNE

Numero de la demande

EP 90 42 0179

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DD-A- 250 451 (SCHERING AG)<br>* revendications *<br>--- | 1 | C 07 C 323/07<br>C 07 C 317/10<br>C 07 D 213/32<br>A 01 N 31/04<br>A 01 N 41/10<br>A 01 N 43/40 |
| A | EP-A-0 157 712 (RHONE-POULENC AGROCHEMIE)<br>* revendication 1 *<br>--- | 1 | |
| A | GB-A-2 197 313 (IMPERIAL CHEMICAL INDUSTRIES PLC)<br>* revendication 1 *<br>--- | 1 | |
| A | GB-A-2 069 492 (J. WYETH & BROTHER)<br>--- | 1 | |
| A | CHEMICAL ABSTRACTS<br>vol. 81, 8-29 juillet 1974, page 271, abrégé no. 12729e, Columbus, Ohio, US; G.H. SCHMID et al.: "Reactions of sulfenyl chlorides and their derivatives. X. Rates and products of addition of 4-chlorobenzenesulfenyl chloride to a series of side chain methyl substituted styrenes." & Can. J. Chem. 1974, vol. 52, no. 9, pages 1807-1812<br>----- | 1 | |

DOMAINES TECHNIQUES RECHERCHES (Int. Cl.5)

C 07 C 323/07
C 07 C 317/10
C 07 D 213/32
A 01 N 31/04
A 01 N 41/10
A 01 N 43/40

Le présent rapport a été établi pour toutes les revendications

| Lieu de la recherche | Date d'achèvement de la recherche | Examinateur |
|---|---|---|
| BERLIN | 05-07-1990 | KAPTEYN H G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant